# EUROPEAN PATENT SPECIFICATION

(11) **EP 1 742 602 B1**
(45) Date of publication and mention of the grant of the patent: **27.06.2012**
(21) Application number: 05742877.3
(22) Date of filing: 04.05.2005
(51) Int. Cl.: A61F 13/15

(54) **SKIN FRIENDLY DIAPER**
HAUTFREUNDLICHE WINDEL
COUCHE DOUCE POUR LA PEAU

(30) Priority: 07.05.2004 US 841119; 04.08.2004 US 911415
(43) Date of publication of application: 17.01.2007
(73) Proprietor: First Quality Products, Inc., State Colllege, PA 16801 (US)
(72) Inventor: KARAMI, Hamzeh, Brewster, MA 02631 (US); DAMAGHI, Babak, Kings Point, NY 11024 (US)
(74) Representative: Cohausz & Florack
(86) International application number: PCT/US2005/015600
(87) International publication number: WO 2005/110310

(56) References cited:
- EP-A- 1 040 800
- WO-A-96/25133
- US-A- 5 718 698
- US-A- 5 928 212
- US-A1- 2004 064 126
- US-B1- 6 307 120

## Description

### FIELD OF THE INVENTION

The present invention relates to an absorbent article comprising a backsheet having a shape defining a longitudinal axis, a minimum lateral dimension, a maximum lateral dimension and at least one aperture therein; one or more hook fasteners, each of said hook fasteners permanently attached at one end to a first part of said backsheet and having a hook fastening material at a second end thereof, said hook fastening material adapted to removably engage a second part of said backsheet; and a containment assembly having a shape defining a maximum lateral dimension, said containment assemble comprising a topsheet; a fluid impermeable backing disposed beneath said topsheet, said fluid impermeable backing formed of a film that resists engagement by said fastening material; and an absorbent core sandwiched between said topsheet and said fluid permeable backing.

### BACKGROUND OF THE INVENTION

Infants and other incontinent individuals wear disposable absorbent artic such as diapers to absorb and contain body exudates discharged from the body, particularity urine. Absorbent articles function to contain the discharged materials in isolation from the body of the wearer on one side, and from the wearers garments and/or bedding on the other. Absorbent articles are well known in the art and are typically constructed from a combination of liquid and vapor pervious and impervious materials which respectively allow the passage of liquid into the diaper and prevent its exit therefrom.

It is known to fasten a diaper about the body of a user using a variety of fasteners having a fastening material such as adhesive or a two part hook-and-loop type (i. e. Velcro). These fasteners are typically located at a front or rear portion of the diaper, such as a flap or wing, and are oriented to engage a "landing zone" on an opposing portion of the diaper. For an adhesive fastener, a release strip may be used as a landing zone; a Velcro fastener requires a special looped landing zone.

US 2003/0004490 Al, issued to Larsson et al. discloses an absorbent article such as a diaper having a landing zone arranged on the front or rear portion of the product and at least one hook-bearing tab arranged on the front or rear portion of the product and at least one hook-bearing tab for detachable interaction with the landing zone. The landing zone includes both active areas, to which the tabs can be fastened, and inactive areas which will not adhere to the tabs. Particularly, the landing zone is a continuous support strip with an inactive area connected between two active areas. This enables two landing zones to be formed in one manufacturing step, from a single strip.

It is also known to make a diaper having Velcro-like hooks as one component of a fastening system and a nonwoven outer surface which serves as the other component. In such a diaper, the hook does not require a special landing zone having special loops. Instead, the entire outer surface of the diaper or brief can function as a landing zone or the hooks. This is know as a "loopless" fastening system, and provides an increased degree of flexibility in the fitting of a diaper to a person. Such a loopless fastener system is described in US 2003/0220626 Al filed on May 7,2003.

Although such a loopless fastening system is more convenient for the user, there may be a tendency to take advantage of the unlimited landing area provided by loopless fasteners to use diapers that are not properly sized to the wearer. Particularly, diapers hat are too large may still be nominally fitted to an individual due to the ability of the loopless fasteners to gather in the slack created by the oversized diaper. This practice is wasteful as larger diapers are likely to be more expensive, and require more material to manufacture.

US 5,387,208 issued to Ashton et al. on February 7,1995 discloses an example of a diaper employing a plurality of layers of pervious, absorbent and impervious materials. Particularly, Ashton et al. discloses a pervious body facing top sheet and impervious garment facing backsheet sandwiching a plurality of layers of variously liquid pervious and absorbent material. The liquid impervious Backsheet extends beyond the dimension of the top and intervening layers, thereby providing laterally extending tabs which can be joined about the waist of the wearer to hold the diaper in place during use.

Although such backsheets do prevent liquid from pasting through the diaper, the impervious nature of tbe backsheet, often a polyethylene film, also prevents the passage of air and water vapor, resulting in a diaper which can feel hot and uncomfortable to wear.

Backsheet which are pervious to vapor are generally known as breathable backsheets and have been described in the art. In general, these backsheets are intended to allow the passage of vapor through them while retarding the passage of liquid. For example, US 3,156,242 issued to Crowe, Jr. on November 10,1964 teaches the use of microporous film as a breathable backsheet. US3,881,489 issued to Hartwell on May 6,19 75 teaches a breathable backsheet having two layers, the first of which is a thermoplastic film and the second of which is a hydrophobic tissue.

While perforated backsheets may provide improved breathability over an impervious backsheet, the materials are of limited utility as they may require multiple layers of materials to prevent leakage. Fundamentally, perforation of otherwise impervious films achieves a measure of breathability at the expense of the material's ability to resist the flow of liquid, particularly when a diaper is subjected to the normal forces created by the wearer during use.

A modified approach is disclosed in US 5,628,737 issued to Dobrin et al. on May 13,1997, which provides a diaper having an impervious backsheet which extends laterally beyond the dimensions of the absorbent core and top sheet on the diaper wherein only the side panels are provided with perforation, thereby providing an impervious region adjacent to the core and a breathable region which permits some movement of vapor therethrough. This approach creates a zone of liquid impermeability where leaks would otherwise be most likely to occur in the backsheet, and provides a breathable region where leaks are less likely, e. g. where the backsheet comes in direct contact with the skin of the wearer. Although the creation of zones of permeability in a diaper resolves some of the problems which are inherent to the backsheets of the prior art, the perforation of even an isolated region of a plastic film backsheet presents its own shortcomings, particularly due to the inherently impervious character of plastic film. For example, an impervious side panel having relatively large or many perforations may achieve the desired breathability, at the expense of the material strength in the perforated zone. Conversely, side panels having relatively few or small perforations may remain strong, yet provide insufficient breathability to ensure the comfort of the wearer.

Basically, the shortcomings of the prior art stem from the attempt to make an impervious material selectively behave like a pervious material. Particularly, when this attempted on a plastic film, the result cannot be accomplished without undermining the plastic film itself, where increased breathability comes at the expense of the material's desirable properties.

An additional disadvantage of the disposable diapers of the prior art is that extensive use of impervious material, typically plastic films, is environmentally detrimental as these films are known to be non-biodegradable. The introduction of perforations into otherwise impervious films as suggested in the prior art docs not render these substances environmentally friendly. The environmental consequences are above and beyond the other economic disadvantages consequences of present diaper designs, particularly that the use of multiple layers of material and the application of the complex manufacturing techniques necessary in current diaper designs render these approaches more costly than necessary to manufacture and therefore less economical to purchase.

Finally, the use of a plastic film as a backsheet precludes the use of a loopless fastener system because a plastic film does not allow any use of the backsheet as a landing zone for a loopless fastener.

Another approach to creating a disposable absorbent article having breathable side panels is found in the Prevail® version of protective underwear manufactured by First Quality Products, Inc. of McElhhattan, Pennsylvania. The brief comprises a nonwoven pervious backsheet having an absorbent assembly attached thereto. The product crotch areas are provided with elastic bands sandwiched between the backsheet and an additional layer of nonwoven material. Thus, the side panels are generally pervious, althoug breathability is impeded by the multiple laminated nonwoven layers, and the adhesive that laminates them. This construction is similar to the Per-Fit® version of diaper, also manufactured by First Quality Products, Inc. which provides increased breathability in side panels comprising two laminated layers of nonwoven material, and is subject to the same drawbacks.

Therefore a need exists for an absorbent article such as a diaper having fastening system which prevents improper sizing of oversized diapers.

A further need exists for an absorbent article such as a diaper having an absorbent core capable of absorbing and retaining fluids, while maximizing the breathability of the article.

A still further need exists for an absorbent article that minimizes the use of fluid impervious and/or non-biodegradable substances.

Document WO 96/25133 A discloses an absorbent article comprising a backsheet having a shape defining a longitudinal axis, a minimum lateral dimension, a maximum lateral dimension and at least one aperture therein; one or more hook fasteners, each of said hook fasteners permanently attached at one end to a first part of said backsheet and having a hook fastening material at a second end thereof, said hook fastening material adapted to removably engage a second part of said backsheet; and a containment assembly having a shape defining a maximum lateral dimension, said containment assembly comprising a topsheet; a fluid impermeable backing disposed beneath said topsheet, said fluid impermeable backing formed of a film that resists engagement by said fastening material; and an absorbent core sandwiched between said topsheet and said fluid permeable backing. In particular, WO 96/25133 A discloses a mechanical fastening-system in the form of two-part hook and loop fasteners, which fastening system comprises a fastening member with a hook-type fastening element and a corresponding landing member formed by cut-out sections. The drawback of said system is that the degree of flexibility in the fitting of a diaper to a person is small.

Similar absorbent articles are also known from US 5,928, 212, US 5,718,698 and EP 1 040 800 A1.

### SUMMARY OF THE INVENTION.

It is therefore an object of the present invention to provide an absorbent article such as a diaper having a fastening-system which is convenient for a user, and at the same time, prevents improper sizing of oversized diapers.

To achieve the above object according to the present invention there is prodded an absorbent article comprising a single layer backsheet having a shape defining a longitudinal axis, a minimum lateral dimension, a maximum lateral dimension and at least one aperture therein, the backsheet being formed af nonwoven material, one or more hook fasteners, each of said hook fasteners permanently attached at one end to a first part of said backsheets and having a hook fastening material at a second end thereof, said hook fastening material adapted to removably engage a second part of said backsheet, and a containment assembly having a shape defining a maximum lateral dimension which is less than the maximum lateral dimension of said backsheet, said containment assembly comprising a topsheet, a fluid impermeable backing disposed beneath said topsheet, said fluid impermeable backing formed of a film that resists engagement by said fastening material, the film being made up of one or more of the following materials polyethylene, polypropylene, polyester, nylon, polyvinyl chloride and blends thereof, and an absorbent core sandwiched between said topsheet and said fluid impermeable backing, wherein said containment assembly is attached to said backsheet along said longitudinal axis, said fluid impermeable backing completely covers said aperture and is exposed therethrough to define a stay-away-zone that resists engagement by said fastening material, and said backsheet forms a plurality of single-layer breathable regions laterally disposed beyond said containment assembly.

Embodiments of the absorbent article according to the present invention are defined in the dependent claims.

Other features and advantages of the present invention will become readily apparent from the following detailed description, the accompanying drawings and the appended claims.

### BRIEF DESCRIPTION OF THE DRAWINGS

FIG. 1 is a perspective view of an embodiment of an absorbent articles which is not part of the invention.
FIG. 2 is a lateral cross-sectional schematic of the absorbent article of FIG. 1.
FIG. 3 is a perspective view of an absorbent article assembled for use which is not part of the invention
FIG. 4 is a lateral cross-sectional schematic of an embodiment of the absorbent article of the invention, incorporating an integral stay-away-zone.
FIG. 5 is a perspective view of the absorbent article of the embodiment of FIG. 4.

### DESCRIPTION OF EMBODIMENTS SHOWN IN THE DRAWINGS

As used herein, the term "absorbent article" refers to devices which absorb and contain body exudates, and more specifically, refers to devices which are placed against or in proximity to the body of the wearer to absorb and contain the various exudates discharged by the body. The term "disposable" is used herein to describe absorbent articles which are not intended to be laundered or otherwise restored or reused as an absorbent article, but instead are intended to be discarded after a single use and, preferably, to be recycled, composed or otherwise disposed of in an environmentally compatible manner. A "unitary" absorbent article refers to absorbent articles, such as diapers, which are formed of separate parts united together to form a coordinated entity so that they do not have multiple parts or require assembly prior to use such as a separate holder and liner.

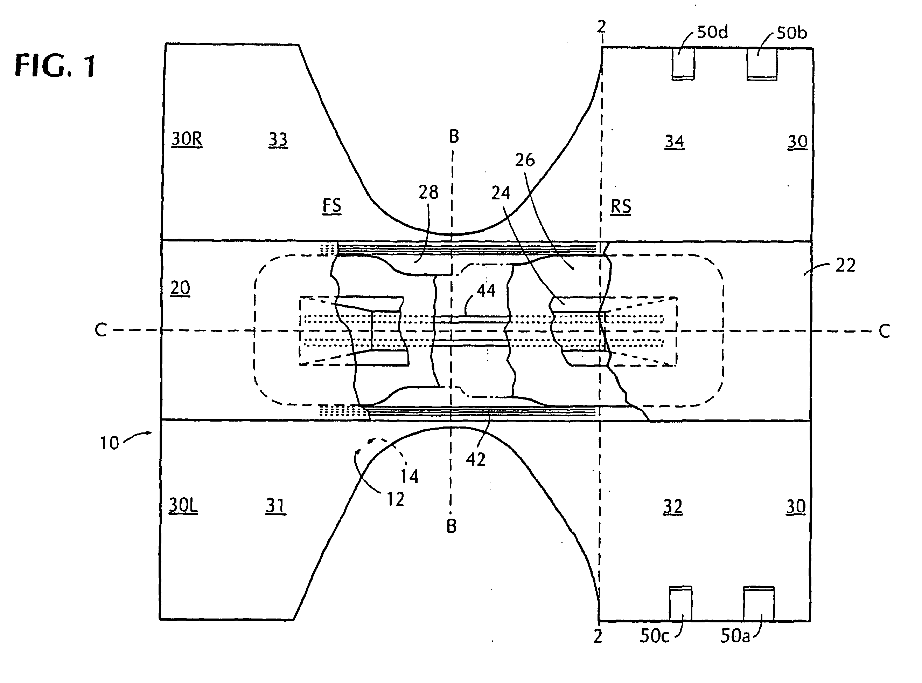

A preferred embodiment of an absorbent article which is not part of the present invention is the unitary disposable diaper 10, shown in FIG. 1. As used herein, the term "diaper" refers to an absorbent article generally worn by infants and incontinent persons that is worn about the lower torso of the wearer. It should be understood, however that the present invention is also applicable to other absorbent articles such as incontinent briefs, incontinent undergarments, training pants, diaper holders, and panty liners and other feminine hygiene products. In particular, training pants have side panels which are pre-attached whereas ordinary diapers have wings which are fastened together to secure the diaper.

FIG. 1 is a plan view of the diaper 10 which is not part of the present invention, with elastic induced contraction pulled out, with a portion of the structure cut away to reveal the inner construction of diaper 10, and with body-facing side 12 facing upwardly Diaper 10 has a longitudinal axis defined by longitudinal centerline C, the term "longitudinal", as used herein, referring to a line, axis or direction in the plane of diaper 10 that is generally aligned with, or parallel to, longitudinal centerline C and defines the length of diaper 10. Transverse axis B extends through diaper 10, intersecting longitudinal centerline C at a right angle in the plane of diaper 10. Transverse axis B defines the transverse orientation relative to diaper 10 and divides diaper 10 into front and rear sections FS and RS respectively. As used herein, the term "transverse" refers to a line, axis or direction that is generally perpendicular to the longitudinal direction and defines the width of diaper 10.

The perimeter of Diaper 10 is defined by backsheet 30. The diaper 10 can be divided into three regions: a containment assembly 20 which extends symmetrically along longitudinal centerline C, and two longitudinally disposed portions 30L and 30R which extend variably in the transverse direction along their length and which define the left and right sides of the diaper respectively. In its preferred embodiment, backsheet 30 of diaper 10 has an "hourglass" configuration wherein portions 30L and 30R narrow to form a crotch region at transverse axis B between front and rear sections FS and RS.

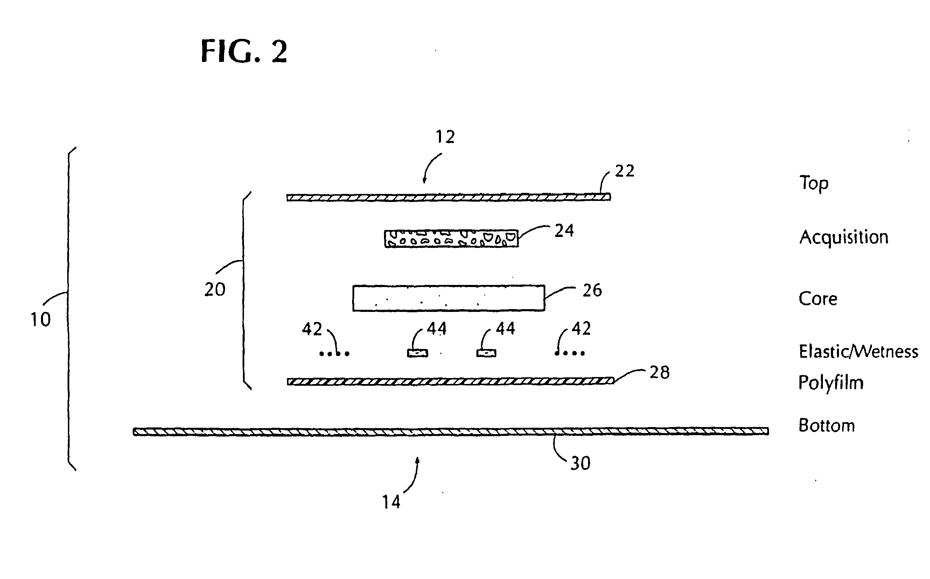

Referring to FIGS. 1 and 2, the structure of diaper 10 is illustrated from body-facing surface 12 toward garment or outer surface 14. Containment assembly 20 preferably comprises a topsheet 22, an acquisition layer 24, an absorbent core 26 and a backing film 28. In a preferred embodiment, crotch elastic bands 42 and/or wetness indicators 44 may be added to one of the components of containment assembly 20. Containment assembly 20 is mounted upon backsheet 30 to form diaper 10.

Topsheet 22 may be made of any suitable relatively liquid-pervious material currently known in the art or later discovered that permits passage of a liquid therethrough. Examples of suitable top sheet materials include nonwoven, spun-bonded or carded webs of polypropylene, polyethelene, nylon, polyester and blends of these materials perforated, apertured or reticulated films, and the like. Nonwoven materials are exemplary because such materials readily allow the passage of liquids to the underlying acquisition layer 24, and therethrough to absorbent core 26. The top sheet is preferably formed of a single ply of nonwoven material that may be made of thermally bonded, spunbonded fibers, spunbond-meltblown-spunbond or fibers that have been hydroentangled, having a basis weight of 10-30 grams per square meter and having appropriate strength and softness for use as a topsheet in an application which will be in contact with human skin. Topsheet 22 may be treated with surfactant, rendering it hydrophilic to facilitate the passage of moisture through topsheet 22 and into the interior of containment assembly 20. The present invention is not intended to be limited to any particular material for top sheet 24 and other top sheet materials will be readily apparent to those skilled in the art.

Acquisition layer 24 may be a single layer or multiple layers made of synthetic or natural material, or a combination of both, or a single multilayer apertured film. Acquisition layer 24 serves to quickly collect and distribute discharged body fluid to absorbent core 26. Because such fluid is typically discharged in gushes, the area of absorbent core 26 proximate to the point of fluid discharge may be overwhelmed by its rate, resulting in a leak. Therefore, the acquisition layer 24 facilitates transport of the fluid from the point of discharge across its surface area to contact other parts of absorbent core 26 from which it can be more readily absorbed. The use of an acquisition layer is well known in the art. Accordingly, acquisition layer 24 of diaper 10 which is not part of the present invention may have any well known or as yet undiscovered composition. Alternatively, absorbent core 26 may have the construction disclosed in US 6,068,620 or 6,646,180 to Chmielewski..

Absorbent core 26 may be any absorbent material which is generally compressible, conformable to the shape of the wearer's body and will not impede normal movement by the wearer, and capable of absorbing and retaining liquids such as urine and certain other body exudates. The absorbent core 26 may be manufactured in a wide variety of sizes and shapes, (e.g., rectangular, hourglass, "T"- shaped, asymmetric, etc.) and from a wide variety of liquid-absorbent materials commonly used in disposable diapers and other absorbent articles such as wood pulp fluff. Examples of other suitable absorbent materials include creped cellulose wadding; meltblown polymers; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of absorbent core 26 may also be varied (e.g., the absorbent core may have varying caliper zones, a hydrophilic gradient, an absorbent gelling material gradient, or lower average density and lower average basis weight acquisition zones; or may comprise one or more layers or structures, i.e., members, including sheets or webs. In addition, each member need not be formed of a single unitary piece of material, but may be formed of a number of smaller strips or components joined together lengthwise or width-wise, as long as they are in fluid communication with one another.) The total absorbent capacity of absorbent core 26 should, however, be compatible with the design loading and the intended use of the diaper 10. Further, the size and absorbent capacity of the absorbent core 26 may be varied to accommodate wearers ranging from infants through adults.

Backing film 28 preferably is made from any suitably pliable liquid impervious material known in the art. Typical backing film materials include films of polyethylene, polypropylene, polyester, nylon and polyvinyl chloride and blends of these materials. For example, backing film 28 can be made of a polyethylene film having a thickness in the range of 0,0127 to 0,0508 mm (0.5 to 2.0 mils). Backing film 28 preferably has sufficient liquid imperviousness to prevent any leakage of fluids. The required level of liquid imperviousness may vary between different locations on diaper 10. Accordingly, the backing film 28 may be made vapor pervious or multi layered, having varying degrees of liquid-imperviousness. Backing film 28 may have the same width as topsheet 22, or may be narrower or wide. Preferably, topsheet 22 and backing film 28 have about the same widths. Backing film 28 may be a composite of a film and another fibrous woven or nonwoven that is, for example, spunbond, melt blown, spunbond-meltblown-spunbond, thermally bonded or chemically bonded. These nonwovens may have very light to moderate bonding. For example, the cross direction tensile strength of each nonwoven may be ess than 118,11 grams/cm (300 grams/inch).

As discussed above, topsheet 22, acquisition layer 24, absorbent core 26 and backing film 28 form the basic components necessary to the preferred embodiment of containment assembly 20. Crotch elastic bands 42 may be adhered to the lateral margins of containment assembly 20 to bias containment assembly 20 into a shape which conforms to that of the wearer's body. Furthermore, wetness indicators 44 may be provided in contact with absorbent core 26 to provide a visual indication that diaper 10 has received and is holding liquid.

Containment assembly 20 may be self contained, for example by adhering the perimeter of topsheet 22 to the perimeter of backing film 28, such as with ordinary adhesive, or by bonding, with heat or ultrasonically, the components to each other. In a construction, acquisition layer 24 and absorbent core 26 are contained within a package formed by backing film 28 and topsheet 22. Containment assembly 20 may then adhered to backsheet 30. Alternatively, topsheet 22 may be adhered directly to backsheet 30, topsheet 22 securing the components of containment assembly 20 between backsheet 30 and topsheet 22. Although the precise shape of containment assembly 20 may vary, it is preferred that top sheet 22 and backing film 28 have generally the same shape and dimensions. Particularly, however, backing film 28 should be sufficiently large to at least completely cover the outer surface of absorbent core 26 to prevent leakage of fluid absorbent core 26 to backsheet 30, but must not significantly exceed tho width of topsheet 22, and should generally be slightly narrower than topsheet 22,

Backsheet 30 is made of a liquid and/or vapor-pervious material which may be selected from the same group of materials from which the top sheet was selected and preferably having a weight of between 5-45 grams per square meter. Unlike topsheet 22, however, the material used for backsheet 30 is preferably rendered hydrophobic by omitting the surfactant discussed above with respect to topsheet 22. Backsheet 30 may be manufactured by well known methods such as thermal bonding, chemical bonding, spun bonding and hydroentanglement.

Backsheet 30 preferably has the same or greater longitudinal dimension to that of containment assembly 20. As discussed below, however, it is critical that backsheet 30 be formed of a single layer of material, and that the lateral dimension of the backsheet exceed, at least in part, the width of containment assembly 20.

As shown in FIG. 1, containment assembly 20 is preferably attached to backsheet 30 symmetrically along longitudinal centerline C. As containment assembly 20 is necessarily narrower and preferably shoter than at least some portions of backsheet 30, portions of backsheet 30 remain unattached from containment assembly 20. In the preferred embodiment, the hourglass shape of backsheet 30 results in two wings in each of longitudinal portions L and R to which containment assembly 20 is not attached.

Alternatively, a T-shaped backsheet would result in one such wing in each of L and R respectively. Wings 31 and 33 are on front section FS of diaper 10 and wings 32 and 34 on rear section RS thereof. As each of wings 31-34 are formed of marginal portions of backsheet 30, they comprise a single layer of liquid and vapor pervious material, rear wings 32 and 34 being provided with fasteners 50a-50d. The fasteners are of the hook-and-loop-type.

A hook-and-loop type arrangement requires fasteners 50a-50d to have a plurality of either hooks or loops disposed on one side thereof, with a landing zones providing corresponding loops or hooks located respectively in corresponding regions on the opposite side of the diaper. The need for landing zones is a result of the fact that nonwoven material may not form a sufficiently strong bond with adhesive or other conventional fasteners to support the assembled diaper during use.

The need to provide a landing zone has been eliminated, however, by the introduction of minibook fasteners which are capable of fastening securely to conventional nonwovens without a corresponding landing zone. These loopless fasteners are ideal for providing a degree of flexibility and choice to the user in the positioning of the fasteners on the diaper.

A problem recognized with the loopless fastener, however, is that the flexibility they provide encourage the use of inappropriately sized diapers, for example a large diaper on a medium sized person, with the fasteners merely being secured to a more remote portion of the nonwoven outer surface of the diaper. This type of misuse is and could not occur in diapers requiring a landing zone as the landing zones limit the areas on the diaper to which a fastener can be applied.

To ameliorate this type of misuse, a stay away zone 100 is provided on portion of the outer surface of the diaper. The stay away zone could be limited to the most remote areas, thereby permitting a wide range of fastener placement while preventing improper sizing of the diaper.

Stay away zone 100 can be created by spraying a solution or attaching a film over a portion of nonwoven backsheet 30 to which the fastener could not attach. Alternatively, a stay away zone could be defined by selectively modifying areas of nonwoven backsheet 30, such as by heat or compression, to destroy its ability to adhere to the hooks of a loopless fastener. For example, a patch having dimensions of 17,78 cm x 27,94 cm (7 inches x 11 inches) and made from stay away zone material may be attached to the outer surface of the front of the diaper to prevent misuse.

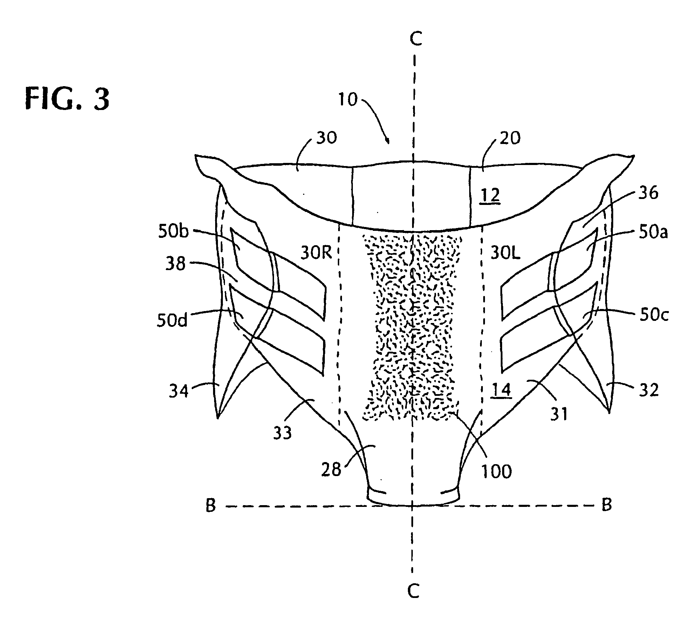

As shown in FIG. 3, diaper 10 is shown as assembled. The diaper 10 is folded about lateral axis B as it would be about a wearer, such that body facing side 12 is oriented inward and outer surface 14 is oriented outward. Containment assembly 20 is shown between left and right longitudinal portions L and R which define the breathable portions of diaper 10 respectively. Specifically, on the left, wing 32 is shown folded over wings 31 and fastened thereto by fasteners 50a and 50c, creating overlapping region 36. Similarly, wing 34 is shown folded over wing 33 and fastened thereto by fasteners 50b and 50d, creating overlapping region 38. Thus, at the regions of diaper 10 defined by longitudinal portions L and R, only a single layer of liquid and vapor permeable material contact the skin of the wearer, with the exception of overlapping regions 36 and 38, which are similarly permeable. Thus, a diaper 10 is provided having breathable sides which increase the comfort of the diaper to the wearer.

Additionally, due to the reduced size of impervious backing film 28 relative to backsheet 30, a reduced amount of film material is required in the construction of diaper 10 relative to a diaper having an impervious backsheet. As backing film 28 is typically plastic and therefore non-biodegradable, the present invention provides a diaper with a minimum of such material.

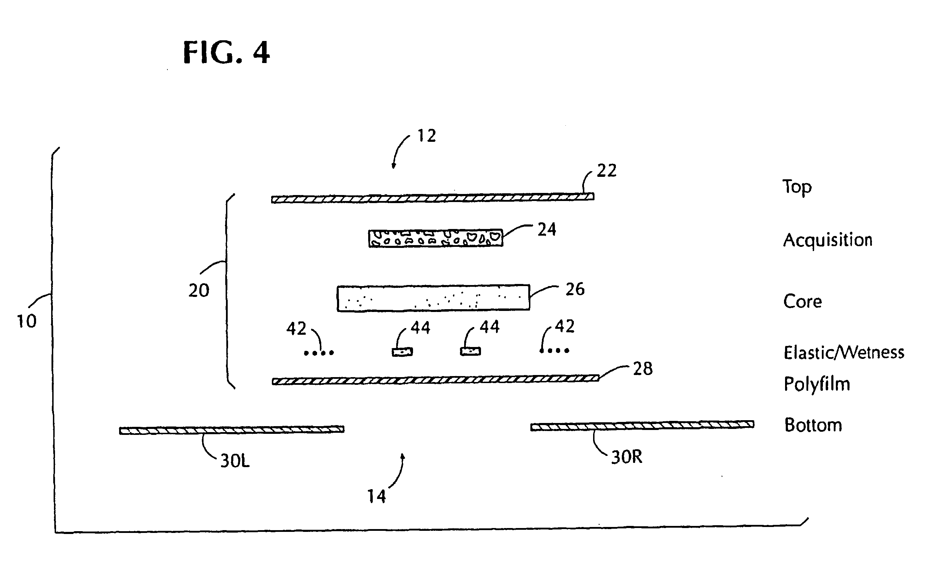

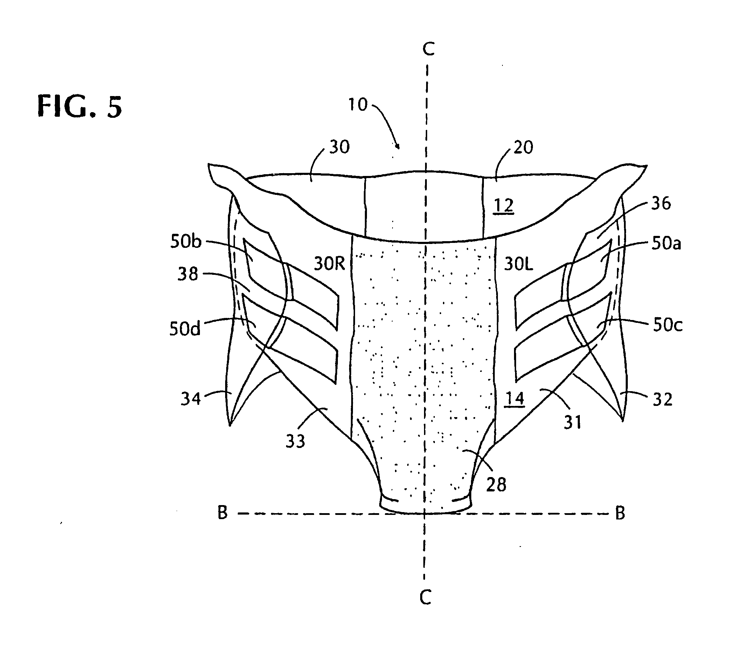

An embodiment of diaper 10 according to the present invention is shown in FIG. 4. The structure of the diaper is essentially the same as that shown in FIG. 2, with the exception that in this embodiment, portions of backsheet 30L and 30R are divided by an aperture which exposes backing film 28 through the backsheet. As shown in FIG. 5, the assembled diaper 10 is illustrated having a region between portions 30R and 30L of backsheet 30 in which backing film 28 is exposed.. The backsheet 30 can be made of any suitable material, such as, for example, spunbond, chemical, thermal bond or hydro entangled (HEF) nonwoven. The aperture dividing the portions of the backsheet 30L and 30R may have any suitable shape, forum and size. For example, the aperture may be rectangular, square, circular, or elliptical and the size of the aperture may be, for example, 5,08-20,32 cm long and 10,16 - 50,8 cm wide (2-8 inches long and 4-20 inches wide), and preferably 10,16 X 30,48 cm(4 X 12 inches) and 10,16 X 35,56 cm(4 X 14 inches) for a large diaper and an extra large diaper, respectively.

FIG. 5 illustrates a preferred embodiment of the stay-away zone wherein the stay-away-zone extends along the entire longitudinal axis of the diaper 10. In such an embodiment, portions 30L and 30R of backsheet 30 do not contact each other on the diaper, and are therefore essentially two separate strips of material defining an aperture between them. In this embodiment, portions 30L and 30R may be attached respectively to the lateral margins of backing film 28 such as by an adhesive or heat-bonding.

The size of the stay-away-zone created by the exposed backing film 28 is limited by the size of the aperture in backsheet 30, which may not extend outside the perimeter of backing film 28. As shown in FIG. 5, the stay-away zone may extend laterally along the entire diaper 10, essentially dividing backsheets 30 into two separate portions 30R and 30L, Likewise, the Location of the stay-away-zone created in this embodiment is inherently limited by the location of backing film 28. In this regard, FIG, 5 illustrates a stay away zone centered about longitudinal centerline C.

Ideally, in cases where the size and location of a stay-away-zone is within the parameters of existing backing film 28, the embodiment of FIGS. 4 and 5 is advantageous as less maternal is needed for backsheet 30, and no additional material or treatment is necessary to create a stay-away-zone on diaper 10. These advantages result in a product of lower cost, and greater environmental benefit Alternatively, however, the shape, location and size of backing film 28 can be modified somewhat, provided that the integrity of containment assembly 20 is not compromised and that backing film 28 does not extend sufficiently into the wings of backsheet 30 to render the sides of diaper 10 liquid and/or vapor impervious.

## Claims

1. An absorbent article comprising:
a single layer backsheet (30) having a shape defining a longitudinal axis (C), a minimum lateral dimension, a maximum lateral dimension and at least one aperture therein, the backsheet (30) being formed of nonwoven material;
one or more hook fasteners (50), each of said hook fasteners (50) permanently attached at one end to a first part of said backsheet (30) and having a hook fastening material at a second end thereof, said hook fastening material adapted to removably engage a second of said backsheet (30) ;
and a containment assembly (20) having a defining a maximum lateral dimension which is less than the maximum lateral dimension of said backsheet(30), said containment assembly (20) comprising:
a topsheet (22);
a fluid impermeable backing (28) disposed beneath said topsheet (22), said fluid impermeable backing (28) formed of a film (28) that resists engagement by said fastening material, the film (28) being made up of one or more of the following materials polyethylene, polypropylene, polyester, nylon, polyvinyl and blends thereof; and
an absorbent core (26) sandwiched between said topsheet (22) and said fluid impermeable backing (28);
wherein said containment assembly (20) is attached to said backsheet (30) along said longitudinal axis (C), said fluid impermeable backing (28) completely covers said aperture and is exposed therethrough to define a stay-away-zone that resists engagement by said fastening material, and said backsheet (30) forms a plurality of single-layer breathable regions laterally disposed beyond said containment assembly (20).

2. The absorbent article of claim 1, wherein said shape of said containment assembly (20) is symmetrical about a longitudinal axis.

3. The absorbent article of claim 2, wherein said topsheet (22) of said containment assembly (20) is attached to said fluid impermeable backing (28), and said absorbant core (26) and said fluid impermeable backing (28) are positioned between said topsheet (22) and said backsheet (30).

4. The absorbent article of claim 2, wherein said topsheet (22) of said containment assembly (20) is attached to at least a portion of said fluid impermeable backing (28).

5. The absorbent article of claim 2, wherein said containment assembly (20) incorporates absorbent members to assist in the placement of said absorbent article against the body of a wearer.

6. The absorbent article of claim 2, wherein an acquisition layer (24) is positioned between said topsheat (22) and said fluid impermeable backing (28) to facilitate distribution of moisture across said absorbent core (26).

7. The absorbent article of claim 1, wherein said backsheet (30) is formed of a spunbond nonwoven and said hook fasteners (50) are removably attachable thereto without adhesive.

8. The absorbent article of claim 7, wherein said backsheet (30) defines a front region at one end of said ; longitudinal axis (C) and a rear region at the other end of said longitudinal axis (C), and a crotch region joining said front and rear regions, wherein said front and rear regions have a lateral dimension of said maximum lateral dimension, and said crotch region has a lateral dimension of said minimum lateral dimension.

9. The absorbent article of claim 8, wherein said maximum lateral dimension of said containment assembly (20) does not exceed the minimum lateral dimension of said backsheet (30).

10. The absorbent article of claim 9, wherein said hook fastening material is adapted to directly engage nonwoven material.

11. The absorbent article of claim 10, wherein said-backsheet (30) is formed of a hydroentangled, spunbond nonwoven.

12. The absorbent article of claim 10, wherein said backsheet (30) is a spunbond nonwoven.

13. The absorbent article of claim 1, wherein said aperture extends along the longitudinal axis (C) of said article, said backsheet (30) being separated into two unconnected portions (30L, 30R).

14. The absorbent article of claim 13, wherein said unconnected portions (30L, 30R) of said backsheet (30) are attached to said fluid impermeable backing (28) by adhesive.

15. The absorbent article of claim 1, wherein said backsheets (30) is one from a group consisting of thermally bonded, chemically bonded, spunbond and hydroentangled nonwoven .

## Patentansprüche

1. Absorbierender Gegenstand, enthaltend:
eine einschichtige Unterschicht (30) mit einer Form, die eine Längsachse (C), eine minimale seitliche Ausdehnung, eine maximale seitliche Ausdehnung und mindestens eine Öffnung darin definiert, wobei die Unterschicht (30) aus Vliesmaterial gebildet ist,
einen oder mehrere Häkchenverschlüsse (50), wobei jeder der Häkchenverschlüsse (50) an einem Ende dauerhaft an einem ersten Teil der Unterschicht (30) angebracht ist und an seinem zweitem Ende ein Häkchenbefestigungsmaterial aufweist, wobei das Häkchenbefestigungsmaterial dafür eingerichtet ist, lösbar in einen zweiten Teil der Unterschicht (30) einzugreifen,
und eine Umhüllungsanordnung (20) mit einer Form, die eine maximale seitliche Ausdehnung definiert, die kleiner als die maximale seitliche Ausdehnung der Unterschicht (30) ist, wobei die Umhüllungsanordnung (20) enthält:
eine Oberschicht (22),
eine fluidundurchlässige Unterlage (28), die unter der Oberschicht (22) angeordnet ist, wobei die fluidundurchlässige Unterlage (28) aus einer Folie (28) gebildet ist, die dem Eingriff durch das Befestigungsmaterial widersteht, wobei die Folie (28) aus einem oder mehreren der folgenden Materialien hergestellt ist: Polyethylen, Polypropylen, Polyester, Nylon,
Polyvinylchlorid und Mischungen daraus, und
einen absorbierenden Kern (26), der zwischen der Oberschicht (22) und der fluidundurchlässigen Unterlage (28) eingelegt ist,
wobei die Umhüllungsanordnung (20) entlang der Längsachse (C) an der Unterschicht (30) angebracht ist, wobei die fluidundurchlässige Unterlage (28) die Öffnung vollständig abdeckt und durch diese freiliegt, um einen Freihaltebereich zu definieren, der dem Eingriff durch das Befestigungsmaterial widersteht, und wobei die Unterschicht (30) mehrere einschichtige atmungsaktive Bereiche bildet, die seitlich über die Umhüllungsanordnung (20) überstehend angeordnet sind.

2. Absorbierender Gegenstand nach Anspruch 1, wobei die Form der Umhüllungsanordnung (20) über eine Längsachse symmetrisch ist.

3. Absorbierender Gegenstand nach Anspruch 2, wobei die Oberschicht (22) der Umhüllungsanordnung (20) an der fluidundurchlässigen Unterlage (28) angebracht ist und der absorbierende Kern (26) und die fluidundurchlässige Unterlage (28) zwischen der Oberschicht (22) und der Unterschicht (30) angeordnet sind.

4. Absorbierender Gegenstand nach Anspruch 2, wobei die Oberschicht (22) der Umhüllungsanordnung (20) an mindestens einem Abschnitt der fluidundurchlässigen Unterlage (28) angebracht ist.

5. Absorbierender Gegenstand nach Anspruch 2, wobei die Umhüllungsanordnung (20) absorbierende Elemente zur Unterstützung beim Anbringen des absorbierenden Gegenstandes am Körper des Trägers enthält.

6. Absorbierender Gegenstand nach Anspruch 2, wobei zwischen der Oberschicht (22) und der fluidundurchlässigen Unterlage (28) eine Aufnahmeschicht (24) angeordnet ist, um die Verteilung von Feuchtigkeit über den absorbierenden Kern (26) zu erleichtern.

7. Absorbierender Gegenstand nach Anspruch 1, wobei die Unterschicht (30) aus einem Spinnvlies gebildet ist und die Häkchenverschlüsse (50) ohne Klebstoff lösbar daran anbringbar ist.

8. Absorbierender Gegenstand nach Anspruch 7, wobei die Unterschicht (30) an einem Ende der Längsachse (C) einen vorderen Bereich und am anderen Ende der Längsachse (C) einen hinteren Bereich definiert sowie einen Schrittbereich, der den vorderen und den hinteren Bereich verbindet, wobei der vordere und der hintere Bereich eine seitliche Ausdehnung aufweisen, die der maximalen seitlichen Ausdehnung entspricht, und der Schrittbereich eine seitliche Ausdehnung aufweist, die der minimalen seitlichen Ausdehnung entspricht.

9. Absorbierender Gegenstand nach Anspruch 8, wobei die maximale seitliche Ausdehnung der Umhüllungsanordnung (20) die minimale seitliche Ausdehnung der Unterschicht (30) nicht übersteigt.

10. Absorbierender Gegenstand nach Anspruch 9, wobei das Häkchenbefestigungsmaterial dafür eingerichtet ist, direkt in Vliesmaterial einzugreifen.

11. Absorbierender Gegenstand nach Anspruch 10, wobei die Unterschicht (30) aus einem wasserstrahlverfestigten Spinnvlies gebildet ist.

12. Absorbierender Gegenstand nach Anspruch 10, wobei die Unterschicht (30) ein Spinnvlies ist.

13. Absorbierender Gegenstand nach Anspruch 1, wobei sich die Öffnung entlang der Längsachse (C) des Gegenstandes erstreckt, wobei die Unterschicht (30) in zwei nicht verbundene Abschnitte (30L, 30R) unterteilt ist.

14. Absorbierender Gegenstand nach Anspruch 13, wobei die nicht verbundenen Abschnitte (30L, 30R) der Unterschicht (30) durch Klebstoff an der fluidundurchlässigen Unterlage (28) angebracht sind.

15. Absorbierender Gegenstand nach Anspruch 1, wobei die Unterschicht (30) eine aus der Gruppe ist, die aus thermisch gebundenem Vlies, chemisch gebundenem Vlies, Spinnvlies und wasserstrahlverfestigtem Vlies besteht.

## Revendications

1. Article absorbant comprenant :
une simple feuille arrière (30) ayant une forme définissant un axe longitudinal (C), une dimension latérale minimale, une dimension latérale maximale et au moins un orifice pratiqué dedans, la feuille arrière (30) étant formée de matériau non tissé ;
une ou plusieurs attaches à crochet (50), chacune desdites attaches à crochet (50) étant attachée de manière permanente par une première extrémité à une première partie de ladite feuille arrière (30) et comportant un accessoire d'attache par crochet à sa seconde extrémité,
ledit accessoire d'attache par crochet étant apte à s'engager de manière amovible dans une seconde partie de ladite feuille arrière (30),
et un ensemble de confinement (20) ayant une forme définissant une dimension latérale maximale qui est inférieure à la dimension latérale maximale de ladite feuille arrière (30), ledit ensemble de confinement (20) comprenant :
une feuille supérieure (22) ;
un fond imperméable au fluide (28) disposé en-dessous de ladite feuille supérieure (22), ledit fond imperméable au fluide (28) étant formé d'un film (28) qui résiste à l'engagement dudit accessoire d'attachement, le film (28) étant fait d'un ou plusieurs des matériaux suivantes : polyéthylène, polypropylène, polyester, nylon, chlorure de polyvinyle et leurs mélanges ; et
un noyau absorbant (26) pris en sandwich entre ladite feuille supérieure (22) et ledit fond imperméable au fluide (28) ;
ledit ensemble de confinement (20) étant attaché à ladite feuille arrière (30) le long dudit axe longitudinal (C), ledit fond imperméable au fluide (28) couvrant complètement ladite ouverture et étant exposé à travers pour définir une zone de répulsion qui résiste à l'engagement dudit accessoire d'attachement et ladite feuille arrière (30) formant une pluralité de zones respirantes à couche simple disposées latéralement au-delà dudit ensemble de confinement (20).

2. Article absorbant selon la revendication 1, ladite forme dudit ensemble de confinement (20) étant symétrique autour d'un axe longitudinal.

3. Article absorbant selon la revendication 2, ladite feuille supérieure (22) dudit ensemble de confinement (20) étant attachée audit fond imperméable au fluide (28) et ledit noyau absorbant (26) et ledit fond imperméable au fluide (28) étant positionnés entre ladite feuille supérieure (22) et ladite feuille arrière (30).

4. Article absorbant selon la revendication 2, ladite feuille supérieure (22) dudit ensemble de confinement (20) étant attachée à au moins une partie dudit fond imperméable au fluide (28).

5. Article absorbant selon la revendication 2, ledit ensemble de confinement (20) comprenant des éléments absorbants pour aider au positionnement dudit article absorbant contre le corps d'un porteur.

6. Article absorbant selon la revendication 2, une couche d'acquisition (24) étant positionnée entre ladite couche supérieure (22) et ledit fond imperméable au fluide (28) pour faciliter la distribution de l'humidité à travers ledit noyau absorbant (26).

7. Article absorbant selon la revendication 1, ladite feuille arrière (30) étant formée d'un non-tissé filé-lié et lesdites attaches à crochet (50) étant attachées de manière amovible à celui-ci sans adhésif.

8. Article absorbant selon la revendication 7, ladite feuille arrière (30) définissant une zone frontale à une extrémité dudit axe longitudinal (C) et une zone arrière à l'autre extrémité dudit axe longitudinal (C) et une zone fourchue joignant ladite zone frontale et arrière, ladite zone frontale et arrière ayant une dimension latérale équivalant à ladite dimension latérale et ladite zone fourchue ayant une dimension latérale équivalant à ladite dimension latérale minimale.

9. Article absorbant selon la revendication 8, ladite dimension latérale maximale dudit ensemble de confinement (20) n'excédant pas la dimension latérale minimale de ladite feuille arrière (30).

10. Article absorbant selon la revendication 9, ledit accessoire d'attachement à crochet étant apte à s'engager directement dans le matériau non tissé.

11. Article absorbant selon la revendication 10, ladite feuille arrière (30) étant formée d'un non-tissé filé-hydrolié.

12. Article absorbant selon la revendication 10, ladite feuille arrière (30) étant un non-tissé filé-lié

13. Article absorbant selon la revendication 1, ladite ouverture s'étendant le long de l'axe longitudinal (C) dudit article, ladite feuille arrière (30) étant séparée en deux parties non connectées (30L, 30R).

14. Article absorbant selon la revendication 13, lesdites parties non connectées (30L, 30R) de ladite feuille arrière (30) étant attachées audit fond imperméable au fluide (28) par de l'adhésif.

15. Article absorbant selon la revendication 1, ladite feuille arrière (30) venant d'un groupe composé de non-tissé lié thermiquement, lié chimiquement, filé-lié et hydrolié.
